# EUROPEAN PATENT SPECIFICATION

(11) **EP 0 231 860 B1**
(45) Date of publication and mention of the grant of the patent: **15.09.1993**
(21) Application number: 87101102.9
(22) Date of filing: 27.01.1987
(51) Int. Cl.: C01B 33/34, B01J 29/06, B01J 20/18

(54) **New zeolite SSZ-25**
Zeolith SSZ-25
Zéolithe SSZ-25

(30) Priority: 29.01.1986 US 823698
(43) Date of publication of application: 12.08.1987
(73) Proprietor: CHEVRON RESEARCH AND TECHNOLOGY COMPANY, San Francisco, California 94105 (US)
(72) Inventor: Zones, Stacey I., San Francisco, CA 94122 (US)
(74) Representative: Müller-Boré & Partner Patentanwälte

(56) References cited:
- US-A- 4 544 538

## Description

Natural and synthetic zeolitic crystalline aluminosilicates are useful as catalysts and adsorbents. These aluminosilicates have distinct crystal structures which are demonstrated by X-ray diffraction. The crystal structure defines cavities and pores which are characteristic of the different species. The adsorptive and catalytic properties of each crystalline aluminosilicate are determined in part by the dimensions of its pores and cavities. Thus, the utility of a particular zeolite in a particular application depends at least partly on its crystal structure.

Because of their unique molecular sieving characteristics, as well as their catalytic properties, crystalline aluminosilicates are especially useful in such applications as gas drying and separation and hydrocarbon conversion. Although many different crystalline aluminosilicates and silicates have been disclosed, there is a continuing need for new zeolites and silicates with desirable properties for gas separation and drying, hydrocarbon and chemical conversions, and other applications.

Crystalline aluminosilicates are usually prepared from aqueous reaction mixtures containing alkali or alkaline earth metal oxides, silica, and alumina. "Nitrogenous zeolites" have been prepared from reaction mixtures containing an organic templating agent, usually a nitrogen-containing organic cation. By varying the synthesis conditions and the composition of the reaction mixture, different zeolites can be formed using the same templating agent.

According to US-Patent No. 4,544,538 there is known a cristalline zeolite, SSZ-13, which is prepared from organic nitrogen-containing cations derived from 1-adamantamine, 3-quinuclidinol, and 2-exo-aminonorbornane. The zeolite further has a composition as synthesized and in an unhydrous state, in terms of mole ratios of oxides as follows: (0.5 to 1.4)R₂O:(0 to 0.50)M₂O:W₂O₃: (greater than 5)YO₂ wherein M is an alkali metal cation, W is selected from aluminium, gallium, and mixtures thereof, Y is selected from silicon, germanium and mixtures thereof, and R is an organic cation. The SSZ-13 zeolite according to the US-Patent No. 4,544,538 can have a YO₂:W₂O₃ mole ratio greater than about 5:1.

Although most experiments reported as producing nitrogenous zeolites have used fairly simple organic species such as tetraalkylammonium cations or alkylenediamines, several experiments are reported as using more complex organic species. U.S. Patent No. 3,692,470, Ciric, Sept. 19, 1972, discloses preparing ZSM-10 from 1,4-dimethyl-1,4 -diazoniabicyclo [2.2-2] octane. U.S. Patent 3,832,449, Rosinski et al, Aug. 27, 1974, discloses preparing ZSM-12 from the reaction products of alkylene dihalides with complex amines or nitrogen heterocycles. U.S. Patent No. 3,950,496, Ciric, Apr. 13, 1976, discloses preparing ZSM-18 from "tris" ammonium hydroxide (1,3,4,6,7,9-hexahydro-2,2,5,5,8,8-hexamethyl-2H-benzo- [1,2-C:3,4-C':5,6-C''] tripyrolium trihydroxide). U.S.Patent No. 4,018,870, Whittam, Apr. 19, 1977, discloses preparing AG5 and AG6 using nitrogenous basic dyes. And, U.S. Patent No. 4,285,922, Audeh, Aug. 25, 1981, discloses preparing ZSM-5 using 1-alkyl, 4 aza, 1-azonia-bycyclo(2,2,2) octane, 4-oxide halides.

Citation No. 93:66052y, in Volume 93 (1980) of Chemical Abstracts, concerns a Russian Language article by Tsitsihrili et al in Soobsch. Akad. Nauk. Gruz., SSR 1980, 97(3)621-4.

This article teaches that the presence of tetramethylammonium ions in a reaction mixture containing K₂O-Na₂O-SiO₂-Al₂O₃-H₂O promotes the crystallization of chabazite. In the absence of the tetramethylammonium ion in the reaction mixture, phillipsite is obtained. The zeolite obtained by the crystallization procedure has a SiO₂:Al₂O₃ mole ratio of 4.23. The article states that the tetramethylammonium ion has a great influence on the direction of crystallization of the reaction mixture, although it may not even enter into the composition of the zeolites.

I have prepared a family of crystalline aluminosilicate molecular sieves with unique properties, referred to herein as "Zeolite SSZ-25", or simply "SSZ-25", and have found a highly effective method for preparing SSZ-25.

SSZ-25 has a mole ratio of an oxide selected from silicon oxide, germanium oxide, and mixtures thereof to an oxide selected from aluminum oxide, gallium oxide, iron oxide, boron oxide and mixtures thereof in the range of 20 to 200, and having the X-ray diffraction lines of Table 1 below. The zeolite further has a composition, as synthesized and in the anhydrous state, in terms of mole ratios of oxides as follows: (0.1 to 2.0)(Q₂O:(0.1 to 2.0)M₂O:W₂O₃:(20 to 200)YO₂ wherein M is an alkali metal cation, W is selected from aluminum, gallium, iron, boron and mixtures thereof, Y is selected from silicon, germanium and mixtures thereof, and Q is an adamantane quaternary ammonium ion. SSZ-25 zeolites can have a YO₂:W₂O₃ mole ratio in the range of 20 to 200. As prepared, the silica:alumina mole ratio is typically in the range of 30:1 to about 100:1. Higher mole ratios can be obtained by treating the zeolite with chelating agents or acids to extract aluminum from the zeolite lattice. The silica:alumina mole ratio can also be increased by using silicon and carbon halides and other similar compounds. Preferably, SSZ-25 is an aluminosilicate wherein W is aluminum and Y is silicon.

My invention also involves a method for preparing SSZ-25 zeolites, comprising preparing an aqueous mixture containing sources of an adamantane quaternary ammonium ion, an oxide selected from aluminum oxide, gallium oxide, iron oxide, boron oxide and mixtures thereof, and an oxide selected from silicon oxide, germanium oxide, and mixtures thereof, and having a composition, in terms of mole ratios of oxides, falling within the following ranges: YO₂/W₂O₃_{,} 20:1 to 200:1; and Q₂O/YO₂ 0.15:1 to 0.50:1; wherein Y is selected from silicon, germanium, and mixtures thereof, W is selected from aluminum, gallium, iron, boron and mixtures thereof, and Q is an adamantane quaternary ammonium ion; maintaining the mixture at a temperature of at least 100°C until the crystals of said zeolite are formed; and recovering said crystals.

SSZ-25 zeolites, as synthesized, have a crystalline structure whose X-ray powder diffraction pattern shows the following characteristic lines:

**Table 1**

| 2 Θ | d/n | I/Iₒ |
|---|---|---|
| 3.05 | 29.0 | 20 |
| 6.42 | 13.77 | 100 |
| 7.18 | 12.31 | 100 |
| 7.88 | 11.22 | 47 |
| 9.62 | 9.19 | 53 |
| 15.75 | 5.63 | 27 |
| 19.37 | 4.58 | 47 |
| 22.57 | 3.94 | 50 |
| 23.05 | 3.86 | 30 |
| 26.03 | 3.42 | 73 |
| 26.85 | 3.32 | 33 |

Typical SSZ-25 aluminosilicate zeolites have the X-ray diffraction pattern of Tables 3-5.

The X-ray powder diffraction patterns were determined by standard techniques. The radiation was the K-alpha/doublet of copper and a scintillation counter spectrometer with a strip-chart pen recorder was used. The peak heights I and the positions, as a function of 2 ϑ where ϑ is the Bragg angle, were read from the spectrometer chart. From these measured values, the relative intensities, 100I/Iₒ, where Iₒ is the intensity of the strongest line or peak, and d, the interplanar spacing in Angstroms corresponding to the recorded lines, can be calculated. The X-ray diffraction pattern of Table 1 is characteristic of SSZ-25 zeolites. The zeolite produced by exchanging the metal or other cations present in the zeolite with various other cations yields substantially the same diffraction pattern although there can be minor shifts in interplanar spacing and minor variations in relative intensity. Variations in the diffraction pattern can also result from variations in the organic compound used in the preparation and from variations in the silicato-alumina mole ratio from sample to sample. Calcination can also cause shifts in the X-ray diffraction pattern. Notwithstanding these perturbations, the basic crystal lattice structure remains unchanged.

After calcination the SSZ-25 zeolites have a crystalline structure whose X-ray powder diffraction pattern shows the following characteristic lines as indicated in Table 2 below:

**Table 2**

| 2 Θ | d/n | I/Iₒ |
|---|---|---|
| 3.4 | 25.5 | 17 |
| 7.19 | 12.30 | 100 |
| 8.04 | 11.00 | 55 |
| 10.06 | 8.78 | 63 |
| 14.35 | 6.17 | 40 |
| 16.06 | 5.51 | 17 |
| 22.77 | 3.90 | 38 |
| 23.80 | 3.74 | 20 |
| 26.08 | 3.417 | 65 |

SSZ-25 zeolites can be suitably prepared from an aqueous solution containing sources of an alkali metal oxide, an adamantane quaternary ammonium ion, an oxide of aluminum, gallium, iron, boron or mixtures thereof, and an oxide of silicon or germanium, or mixture of the two. The reaction mixture should have a composition in terms of mole ratios falling within the following ranges:

| | Broad | Preferred |
|---|---|---|
| YO₂/W₂O₃ | 20-200 | 30-100 |
| OH⁻/YO₂ | 0.10-1.0 | 0.20-0.40 |
| Q/YO₂ | 0.15-0.50 | 0.15-0.30 |
| M⁺/YO₂ | 0.05-0.30 | 0.15-0.30 |
| H₂O/YO₂ | 20-300 | 35-60 |
| Q/Q+M⁺ | 0.30-0.70 | 0.40-0.67 |

wherein Q is an adamantane quaternary ammonium ion, Y is silicon, germanium or both, and W is aluminum, gallium, iron, boron or mixtures thereof. M is an alkali metal, preferably sodium or potassium. The organic adamantane compound which acts as a source of the adamantane quaternary ammonium ion employed can provide hydroxide ion.

When using the adamantane quaternary ammonium hydroxide compound as a template, it has also been found that purer forms of SSZ-25 are prepared when there is an excess of the adamantane quaternary ammonium hydroxide compound present relative to the amount of alkali metal hydroxide and that when the OH⁻/SiO₂ molar ratio is greater than 0.40, then M⁺/SiO₂ molar ratio should be less than 0.20.

The adamantane quaternary ammonium ion component Q, of the crystallization mixture, is derived from an adamantane quaternary ammonium compound. Preferably, the adamantane quaternary ammonium ion is derived from a compound of the formula 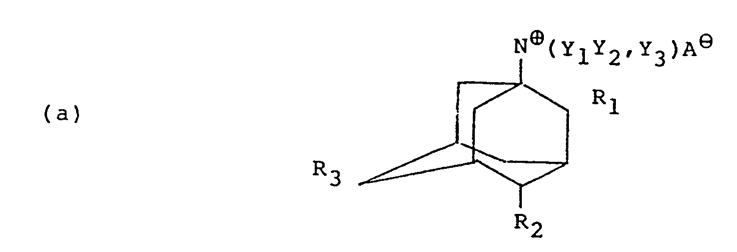
wherein each of Y₁ Y₂ and Y₃ independently is lower alkyl and most preferably methyl; A^{Θ} is an anion which is not detrimental to the formation of the zeolite; and each of R₁, R₂, and R₃ independently is hydrogen, or lower alkyl and most preferably hydrogen; and 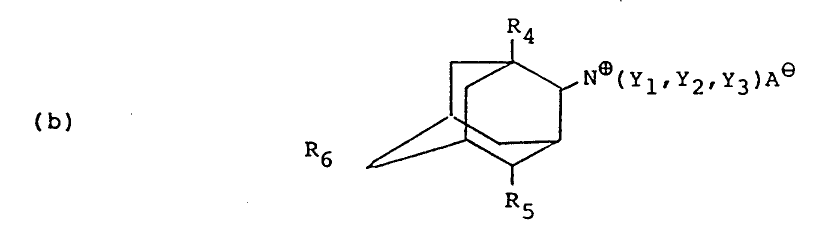
wherein each of R₄, R₅ and R₆ independently is hydrogen or lower alkyl; and most preferably hydrogen; each of Y₁, Y₂ and Y₃ independently is lower alkyl and most preferably methyl; and A^{Θ} is an anion which is not detrimental to the formation of the zeolite;
The adamantane quaternary ammonium compounds are prepared by methods known in the art.

Use of N,N,N-trimethyl cyclopentylammonium iodide in the preparation of Zeolite SSZ-15 molecular sieve is disclosed in U.S. Patent No. 4,610,854; use of 1-azoniaspiro [4.4] nonyl bromide and N,N,N-trimethyl neopentylammonium iodide in the preparation of a molecular sieve termed "Losod" is disclosed in Helv. Chim. Acta (1974); Vol. 57, page 1533 (W. Sieber and W. M. Meier); use of quinuclidinium compounds to prepare a zeolite termed "NU-3" is disclosed in European Patent Publication No. 40016; use of 1,4-di(1-azoniabicyclo [2.2.2.]octane) lower alkyl compounds in the preparation of Zeolite SSZ-16 molecular sieve is disclosed in U.S. Patent No. 4,508,837; use of N,N,N-trialkyl-1-adamantamine in the preparation of zeolite SSZ-13 molecular sieve is disclosed in U.S. Patent No. 4,544,538.

By lower alkyl is meant alkyl of from about 1 to 5 carbon atoms.

A^{Θ} is an anion which is not detrimental to the formation of the zeolite. Representative of the anions include halide, e.g., fluoride, chloride, bromide and iodide, hydroxide, acetate, sulfate, carboxylate, etc. Hydroxide is the most preferred anion. It may be beneficial to ion-exchange, for example, the halide for hydroxide ion, thereby reducing or eliminating the alkali metal hydroxide quantity required.

The reaction mixture is prepared using standard zeolitic preparation techniques. Typical sources of aluminum oxide for the reaction mixture include aluminates, alumina, and aluminum compounds such as AlCl₃ and Al₂(SO₄)₃. Typical sources of silicon oxide include silicates, silica hydrogel, silicic acid, colloidal silica, tetraalkyl orthosilicates, and silica hydroxides. Gallium, iron, boron and germanium can be added in forms corresponding to their aluminum and silicon counterparts. Salts, particularly alkali metal halides such as sodium chloride, can be added to or formed in the reaction mixture. They are disclosed in the literature as aiding the crystallization of zeolites while preventing silica occlusion in the lattice.

The reaction mixture is maintained at an elevated temperature until the crystals of the zeolite are formed. The temperatures during the hydrothermal crystallization step are typically maintained from about 140°C to about 200°C, preferably from about 160°C to about 180°C and most preferably from about 170°C to about 180°C. The crystallization period is typically greater than 1 day and preferably from about 5 days to about 10 days.

The hydrothermal crystallization is conducted under pressure and usually in an autoclave so that the reaction mixture is subject to autogenous pressure. The reaction mixture can be stirred during crystallization.

Once the zeolite crystals have formed, the solid product is separated from the reaction mixture by standard mechanical separation techniques such as filtration. The crystals are water-washed and then dried, e.g., at 90°C to 150°C for from 8 to 24 hours, to obtain the as synthesized, SSZ-25 zeolite crystals. The drying step can be performed at atmospheric or subatmospheric pressures.

During the hydrothermal crystallization step, the SSZ-25 crystals can be allowed to nucleate spontaneously from the reaction mixture. The reaction mixture can also be seeded with SSZ-25 crystals both to direct, and accelerate the crystallization, as well as to minimize the formation of undesired aluminosilicate contaminants. If the reaction mixture is seeded with SSZ-25 crystals, the concentration of the organic compound can be greatly reduced or eliminated, but it is preferred to have some organic compound present, e.g., an alcohol.

The synthetic SSZ-25 zeolites can be used as synthesized or can be thermally treated (calcined). Usually, it is desirable to remove the alkali metal cation by ion exchange and replace it with hydrogen, ammonium, or any desired metal ion. The zeolite can be leached with chelating agents, e.g., EDTA or dilute acid solutions, to increase the silica:alumina mole ratio. The zeolite can also be steamed; steaming helps stabilize the crystalline lattice to attack from acids. The zeolite can be used in intimate combination with hydrogenating components, such as tungsten, vanadium, molybdenum, rhenium, nickel, cobalt, chromium, manganese, or a noble metal, such as palladium or platinum, for those applications in which a hydrogenation-dehydrogenation function is desired. Typical replacing cations can include metal cations, e.g., rare earth, Group IIA and Group VIII metals, as well as their mixtures. Of the replacing metallic cations, cations of metals such as rare earth, Mn, Ca, Mg, Zn, Cd, Pt, Pd, Ni, Co, Ti, Al, Sn, Fe and Co are particularly preferred.

The hydrogen, ammonium, and metal components can be exchanged into the zeolite. The zeolite can also be impregnated with the metals, or, the metals can be physically intimately admixed with the zeolite using standard methods known to the art. And, the metals can be occluded in the crystal lattice by having the desired metals present as ions in the reaction mixture from which the SSZ-25 zeolite is prepared.

Typical ion exchange techniques involve contacting the synthetic zeolite with a solution containing a salt of the desired replacing cation or cations. Although a wide variety of salts can be employed, chlorides and other halides, nitrates, and sulfates are particularly preferred. Representative ion exchange techniques are disclosed in a wide variety of patents including U.S. Nos. 3,140,249; 3,140,251; and 3,140,253. Ion exchange can take place either before or after the zeolite is calcined.

Following contact with the salt solution of the desired replacing cation, the zeolite is typically washed with water and dried at temperatures ranging from 65°C to about 315°C. After washing, the zeolite can be calcined in air or inert gas at temperatures ranging from about 200°C to 820°C for periods of time ranging from 1 to 48 hours, or more, to produce a catalytically active product especially useful in hydrocarbon conversion processes.

Regardless of the cations present in the synthesized form of the zeolite, the spatial arrangement of the atoms which form the basic crystal lattice of the zeolite remains essentially unchanged. The exchange of cations has little, if any, effect on the zeolite lattice structures.

The SSZ-25 aluminosilicate can be formed into a wide variety of physical shapes. Generally speaking, the zeolite can be in the form of a powder, a granule, or a molded product, such as extrudate having particle size sufficient to pass through a 9.8 mm (2-mesh (Tyler)) screen and be retained on a 0.038 mm (400-mesh (Tyler)) screen. In cases where the catalyst is molded, such as by extrusion with an organic binder, the aluminosilicate can be extruded before drying, or, dried or partially dried and then extruded.The zeolite can be composited with other materials resistant to the temperatures and other conditions employed in organic conversion processes. Such matrix materials include active and inactive materials and synthetic or naturally occurring zeolites as well as inorganic materials such as clays, silica and metal oxides. The latter may occur naturally or may be in the form of gelatinous precipitates, sols, or gels, including mixtures of silica and metal oxides. Use of an active material in conjunction with the synthetic zeolite, i.e., combined with it, tends to improve the conversion and selectivity of the catalyst in certain organic conversion processes. Inactive materials can suitably serve as diluents to control the amount of conversion in a given process so that products can be obtained economically without using other means for controlling the rate of reaction. Frequently, zeolite materials have been incorporated into naturally occurring clays, e.g., bentonite and kaolin. These materials, i.e., clays, oxides, etc., function, in part, as binders for the catalyst. It is desirable to provide a catalyst having good crush strength, because in petroleum refining the catalyst is often subjected to rough handling. This tends to break the catalyst down into powders which cause problems in processing.

Naturally occurring clays which can be composited with the synthetic zeolites of this invention include the montmorillonite and kaolin families, which families include the sub-bentonites and the kaolins commonly known as Dixie, McNamee, Georgia and Florida clays or others in which the main mineral constituent is halloysite, kaolinite, dickite, nacrite, or anauxite. Fibrous clays such as sepiolite and attapulgite can also be used as supports. Such clays can be used in the raw state as originally mined or can be initially subjected to calcination, acid treatment or chemical modification.

In addition to the foregoing materials, the SSZ-25 zeolites can be composited with porous matrix materials and mixtures of matrix materials such as silica, alumina, titania, magnesia, silica:alumina, silica-magnesia, silica-zirconia, silica-thoria, silica-beryllia, silica-titania, titania-zirconia as well as ternary compositions such as silica-alumina-thoria, silica-alumina-zirconia, silica-alumina-magnesia and silica-magnesia-zirconia. The matrix can be in the form of a cogel.

The SSZ-25 zeolites can also be composited with other zeolites such as synthetic and natural faujasites (e.g., X and Y), erionites, and mordenites. They can also be composited with purely synthetic zeolites such as those of the ZSM series. The combination of zeolites can also be composited in a porous inorganic matrix.

SSZ-25 zeolites are useful in hydrocarbon conversion reactions. Hydrocarbon conversion reactions are chemical and catalytic processes in which carbon containing compounds are changed to different carbon containing compounds. Examples of hydrocarbon conversion reactions include catalytic cracking, hydrocracking, and olefin and aromatics formation reactions. The catalysts are useful in other petroleum refining and hydrocarbon conversion reactions such as isomerizing n-paraffins and naphthenes, polymerizing and oligomerizing olefinic or acetylenic compounds such as isobutylene and butene-1, reforming, alkylating, isomerizing polyalkyl substituted aromatics (e.g., ortho xylene), and disproportionating aromatics (e.g., toluene) to provide mixtures of benzene, xylenes and higher methylbenzenes. The SSZ-25 catalysts have high selectivity, and under hydrocarbon conversion conditions can provide a high percentage of desired products relative to total products.

SSZ-25 zeolites can be used in processing hydrocarbonaceous feedstocks. Hydrocarbonaceous feedstocks contain carbon compounds and can be from many different sources, such as virgin petroleum fractions, recycle petroleum fractions, shale oil, liquefied coal, tar sand oil, and, in general, can be any carbon containing fluid susceptible to zeolitic catalytic reactions. Depending on the type of processing the hydrocarbonaceous feed is to undergo, the feed can contain metal or be free of metals, it can also have high or low nitrogen or sulfur impurities. It can be appreciated, however, that in general processing will be more efficient (and the catalyst more active) the lower the metal, nitrogen, and sulfur content of the feedstock.

The conversion of hydrocarbonaceous feeds can take place in any convenient mode, for example, in fluidized bed, moving bed, or fixed bed reactors depending on the types of process desired. The formulation of the catalyst particles will vary depending on the conversion process and method of operation.

Other reactions which can be performed using the catalyst of this invention containing a metal, e.g., platinum, include hydrogenation-dehydrogenation reactions, denitrogenation and desulfurization reactions.

SSZ-25 can be used in hydrocarbon conversion reactions with active or inactive supports, with organic or inorganic binders, and with and without added metals. These reactions are well known to the art, as are the reaction conditions.

SSZ-25 can also be used as an adsorbent, as a filler in paper, paint, and toothpastes, and as a water-softening agent in detergents.

The following Examples illustrate the preparation of SSZ-25.

### EXAMPLES

### Example 1

### Preparation of N,N,N-Trimethyl-1-adamantanammonium Hydroxide (Template A)

Ten (10) gram of 1-adamantanamine (Aldrich) was dissolved in a mixture of 29 gram tributylamine and 60 ml dimethylformamide. The mixture was chilled in an ice bath.

28.4 Gram of methyl iodide were added dropwise to the chilled solution with continuous stirring. After several hours crystals appear. The reaction was continued overnight and allowed to come to room temperature. The crystals were filtered and washed with tetrahydrofuran and then diethyl ether before vacuum drying. Additional product was obtained by adding enough diethyl ether to the reaction filtrate to produce two phases and then with vigorous stirring acetone was added until the solution just became one phase. Continued stirring produced crystallization at which time the solution can be chilled to induce further crystallization. The product has a melting point near 300°C (decomp.) and the elemental analyses and NMR are consistent with the known structure. The vacuum-dried iodide salt was then ion-exchanged with ion-exchange resin AG 1x8 (in molar excess) to the hydroxide form. The exchange was performed over a column or more preferably by overnight stirring of the resin beads and the iodide salt in an aqueous solution designed to give about a 0.5 molar solution of the organic hydroxide. This produces Template A.

### Example 2

### Preparation of N,N,N-Trimethyl-2-adamantan-ammonium Hydroxide (Template B)

Five gram of 2-adamantanone (Aldrich Chemical Co.) was mixed with 2.63 gram of formic acid (88%) and 4.5 g of dimethyl formamide. The mixture was then heated in a pressure vessel for 16 hours at 190°C. Care should be taken to anticipate the increase in pressure the reaction experiences due to CO₂ evolution. The reaction was conveniently carried out in a Parr 4748 reactor with teflon liner. The workup consists of extracting N,N dimethyl-2-adamantamine from a basic (pH=12) aqueous solution with diethyl ether. The various extracts were dried with Na₂SO₄ the solvent removed and the product taken up in ethyl acetate. An excess of methyl iodide was added to a cooled solution which was then stirred at room temperature for several days. The crystals were collected and washed with diethyl ether to give N,N,N trimethyl-2-adamantammonium iodide. The product is checked by microanalysis for C, H, and N. The conversion to the hydroxide form was carried out analogously to Template A above.

### Example 3

4.5 Gram of a 0.67 M solution of Template B in its hydroxide form were mixed with 6 ml H₂O and 0.103 g of KOH (solid). After dissolution 2.36 g of Ludox AS-30 colloidal silica (30% SiO₂) were added with stirring using a magnetic stir bar. Finally 0.78 g of Nalco lSJ612 alumina on silica (30% solids, 4% Al₂O₃ overall) was added. The reactants were loaded into a Parr 4745 reactor, sealed and loaded onto a rotating spit in a Blue M oven. The reactor was rotated at 30r⁻¹(RPM) while being heated at 175°C for 10 days. The product after filtration, washing with distilled water, drying in air and then at 100°C was the crystalline material designated SSZ-25. The X-ray diffraction pattern of the as-made material is tabulated in Table 3 below.

**Table 3**

| 2 Θ | d/n | I/Iₒ |
|---|---|---|
| 3.05 | 29.0 | 20 |
| 6.42 | 13.77 | 100 |
| 7.18 | 12.31 | 100 |
| 7.88 | 11.22 | 47 |
| 9.62 | 9.19 | 53 |
| 12.85 | 6.89 | 13 |
| 14.37 | 6.16 | 10 |
| 14.73 | 6.01 | 23 |
| 15.75 | 5.63 | 27 |
| 16.13 | 5.49 | 13 |
| 19.37 | 4.58 | 47 |
| 20.09 | 4.42 | 23 |
| 20.78 | 4.27 | 10 |
| 21.65 | 4.105 | 50 |
| 22.57 | 3.939 | 50 |
| 23.05 | 3.858 | 30 |
| 23.70 | 3.754 | 17 |
| 25.00 | 3.562 | 20 |
| 26.03 | 3.423 | 73 |
| 26.85 | 3.320 | 33 |
| 27.28 | 3.269 | 17 |
| 28.91 | 3.088 | 13 |

### Example 4

6.02 Gram of a 0.71 M solution of Template A were mixed with 0.14 g KOH(s), 0.088 g of Reheis F-2000 hydrated alumina, and 8 ml H₂O. After thorough mixing 4.0 g of Ludox AS-30 was blended in as silica source. The reaction mixture was heated in the teflon cup of a Parr 4745 reactor at 175°C at 45r⁻¹(RPM) for 7 days. Workup as in Example 3 produced crystalline SSZ-25.

### Example 5

In this example the same reactants were used as in Example 4 but the initial SiO₂/Al₂O₃ ratio was increased to 75. 0.051 of Reheis F-2000 hydrated alumina was used and dissolved in the same quantity KOH, 6.4 g of the same Template A solution and 6.8 ml H₂O. The same quantity of Ludox was used and the reaction was again run at 175°C but at 30r⁻¹(RPM). At 7 days of reaction the product was largely amorphous but by 10 days of reaction the product was crystalline SSZ-25. The SiO₂/Al₂O₃ value of the zeolite is 75.

### Example 6

3.00 Gram of a 1.04 M solution of Template A was mixed with 9 ml of H₂O, 0.195 g of KOH(s), 0.083 g of Reheis F-2000 hydrated alumina, and finally 0.90 g of Cabosil M5. The mixture was heated at 175°C for 7 days without agitation. The crystalline product was SSZ-25 and has a SiO₂/Al₂O₃ ratio of 30.

### Example 7

A reaction like Example 4 was set up again. This time a half-inch diameter teflon ball was added to the reactor to aid in mixing during the tumbling of the reactor. The crystalline product after 7 days of reaction and the usual workup was SSZ-25.

### Example 8

The crystalline products of Examples 3-7 were subjected to calcination as follows. The samples were heated in a muffle furnace from room temperature up to 540°C at a steadily increasing rate over a 7-hour period. The samples were maintained at 540°C for four more hours and then taken up to 600°C for an additional four hours. A 50/50 mixture of air and nitrogen was passed over the zeolites at a rate of 400.7m³(20 standard cubic feet) per minute during heating. Representative X-ray diffraction data for the calcined products of Example 3 appears in Table 4.

**Table 4**

| 2 Θ | d/n | I/Iₒ |
|---|---|---|
| 3.35 | 26.4 | 18 |
| 7.18 | 12.31 | 100 |
| 8.00 | 11.05 | 62 |
| 10.04 | 8.80 | 68 |
| 12.90 | 6.86 | 12 |
| 14.33 | 6.18 | 44 |
| 14.80 | 5.99 | 12 |
| 16.01 | 5.53 | 18 |
| 20.31 | 4.37 | 18 |
| 21.70 | 4.10 | 44 |
| 22.73 | 3.91 | 41 |
| 23.80 | 3.74 | 24 |
| 25.02 | 3.559 | 15 |
| 26.06 | 3.420 | 68 |
| 27.02 | 3.300 | 15 |
| 27.89 | 3.999 | 18 |
| 28.71 | 3.109 | 12 |

The SSZ-25 product of Example 4 was also calcined in a muffler furnace at 463°C for a period of 2 hours and the representative X-ray diffraction data for the calcined product appears in Table 5 below.

**Table 5**

| 2 Θ | d/n | I/Iₒ |
|---|---|---|
| 3.5 | 25.2 | 16 |
| 7.20 | 12.28 | 100 |
| 8.07 | 10.96 | 47 |
| 9.68 | 9.14 | 74 |
| 10.08 | 8.77 | 58 |
| 13.05 | 6.78 | 15 |
| 14.37 | 6.16 | 36 |
| 14.82 | 5.98 | 11 |
| 16.10 | 5.50 | 16 |
| 18.03 | 4.92 | 8 |
| 20.30 | 4.37 | 12 |
| 20.95 | 4.24 | 18 |
| 21.66 | 4.10 | 11 |
| 21.97 | 4.05 | 18 |
| 22.80 | 3.900 | 34 |
| 23.80 | 3.739 | 18 |
| 25.03 | 3.558 | 14 |
| 25.32 | 3.517 | 15 |
| 26.10 | 3.414 | 61 |
| 27.05 | 3.296 | 15 |
| 27.95 | 3.192 | 14 |
| 28.71 | 3.109 | 11 |

Considerable changes in peak position and intensity can be observed in comparing the data for SSZ-25 before and after calcination.

### Example 9

Ion-exchange of the calcined SSZ-25 materials from Example 8 was carried out using NH₄NO₃ to convert the zeolites from their K form to NH₄ and then eventually H form. Typically the same mass of NH₄NO₃ as zeolite was slurried into H₂O at ratio of 50/1 H₂O to zeolite. The exchange solution was heated at 100°C for two hours and then filtered. This process was repeated four times. Finally, after the last exchange the zeolite was washed several times with H₂O and dried. A repeat calcination as in Example 8 was carried out but without the final treatment at 600°C. This produces the H form of SSZ-25 zeolite.

### Example 10

The product of Example 6, after sequential treatment as in Examples 8 and then 9, was subjected to a surface area and pore size distribution analysis using N₂ as adsorbate and via the BET method. The surface area of the zeolitic material was 520 m²/g and the micropore volume was 0.18 cm³/g.

### Example 11

### Constraint Index Determination:

0.25 Gram of the hydrogen form of the zeolite of Example 3 (after treatment according to Examples 8 and 9, was packed into a 0.952cm(3/8") stainless steel tube with alundum on both sides of the zeolite bed. A Lindburg furnace was used to heat the reactor tube. Helium was introduced into the reactor tube at 10cm³/min. and atmospheric pressure. The reactor was taken to 121.1°C(250°F) for 40 min. and then raised to 315.6°C(600°F). Once temperature equilibration was achieved a 50/50, w/w feed of n-hexane and 3-methylpentane was introduced into the reactor at a rate of 0.62cm³/hr. Feed delivery was made via syringe pump. Direct sampling onto a gas chromatograph begun after 10 minutes of feed introduction. The constraint index value was calculated from gas chromatographic data using methods known in the art.

| Example No. | C.I. | Conversion at 10 min. | Temp.(°F) °C |
|---|---|---|---|
| 3 | 0.3 | 65% | (600) 315.6 |

## Claims

1. A zeolite having a mole ratio of an oxide selected from silicon oxide, germanium oxide and mixtures thereof to an oxide selected from aluminium oxide, gallium oxide, iron oxide, boron oxide and mixtures thereof greater than about 20:1,
**characterized by**
the following X-ray diffraction lines:
| 2 Θ | d/n | I/Iₒ |
|---|---|---|
| 3,05 | 29,0 | 20 |
| 6,42 | 13,77 | 100 |
| 7,18 | 12,31 | 100 |
| 7,88 | 11,22 | 47 |
| 9,62 | 9,19 | 53 |
| 15,75 | 5,63 | 27 |
| 19,37 | 4,58 | 47 |
| 22,57 | 3,94 | 50 |
| 23,05 | 3,86 | 30 |
| 26,03 | 3,42 | 73 |
| 26,85 | 3,32 | 33 |

2. A zeolite having a composition, as synthesized and in the anhydrous state, in terms of mole ratios of oxides as follows: (0,1 to 2,0)Q₂O:(1,1 to 2,0)M₂O:W₂O₃:-(greater than 20) YO₂ wherein M is an alkali metal cation, W is selected from aluminium, gallium, iron, boron and mixtures thereof, Y is selected from silicon, germanium and mixtures thereof, Q is an adamantane quarternary ammonium ion,
**characterized by**
the following X-ray diffraction lines:
| 2 Θ | d/n | I/Iₒ |
|---|---|---|
| 3,05 | 29,0 | 20 |
| 6,42 | 13,77 | 100 |
| 7,18 | 12,31 | 100 |
| 7,88 | 11,22 | 47 |
| 9,62 | 9,19 | 53 |
| 15,75 | 5,63 | 27 |
| 19,37 | 4,58 | 47 |
| 22,57 | 3,94 | 50 |
| 23,05 | 3,86 | 30 |
| 26,03 | 3,42 | 73 |
| 26,85 | 3,32 | 33 |

3. The zeolite according to Claim 1 or 2 wherein W is aluminum and Y is silicon.

4. A zeolite prepared by thermally treating the zeolite of Claim 3 at a temperature from about 200°C to 820°C.

5. The zeolite according to Claim 4 having the X-ray diffraction lines of Table 2.

6. The zeolite according to Claim 1 or 2 wherein said mole ratio of silicon oxide or germanium oxide to aluminum oxide, gallium oxide, iron oxide or boron oxide is about 20:1 to 200:1.

7. The zeolite according to Claim 1 or 2 wherein said mole ratio of silicon oxide or germanium oxide to aluminum oxide, gallium oxide, iron oxide or boron oxide is about 30:1 to 100:1.

8. A zeolite according to Claim 2 wherein the adamantane quaternary ammonium ion is derived from an adamantane compound of the formula: 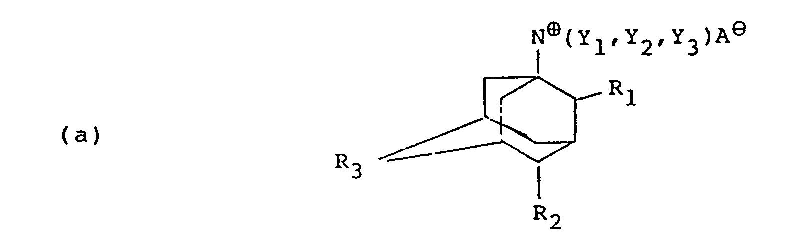 wherein each of Y₁ Y₂ and Y₃ independently is lower alkyl and A^{ϑ} is an anion which is not detrimental to the formation of the zeolite; and each of R₁, R₂ and R₃ independently is hydrogen, or lower alkyl; and 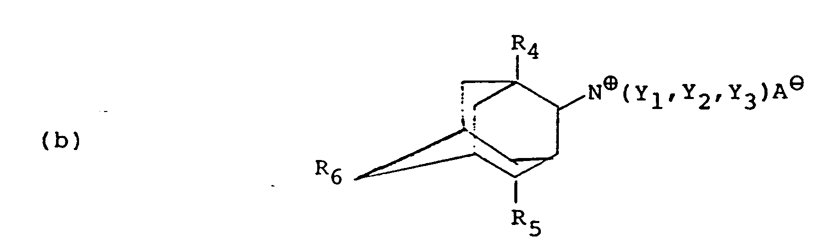 wherein each of R₄, R₅ and R₆ independently is hydrogen or lower alkyl; each of Y₁, Y₂ and Y₃ independently is lower alkyl; and A^{ϑ} is an anion which is not detrimental to the formation of the zeolite.

9. A zeolite according to Claim 8 wherein in formula (a) each of Y₁, Y₂ and Y₃ independently is methyl or ethyl; A^{ϑ} is OH or halogen; and each of R₁, R₂, and R₃ is hydrogen; and in formula (b) each of Y₁, Y₂ and Y₃ independently is methyl or ethyl; A^{ϑ} is OH, or halogen; and each of R₄, R₅ and R₆ is hydrogen.

10. A zeolite according to Claim 9 wherein Y**₁,** Y**₂** and Y₃ are the same and each is methyl; and A^{ϑ} is OH, or I.

11. A zeolite according to Claim 1 or 2 which has undergone ion exchange with hydrogen, ammonium, rare earth metal, Group IIA metal, or Group VIII metal ions.

12. A zeolite according to Claim 1 or 2 wherein rare earth metals, Group IIA metals, or Group VIII metals are occluded in the zeolite.

13. A zeolite composition, comprising the zeolite of Claim 1 or 2 and an inorganic matrix.

14. A method for preparing the zeolite of the preceding claims comprising:
(a) preparing an aqueous mixture containing sources of an adamantane quarternary ammonium ion, an oxide selected from aluminium oxide, gallium oxide, iron oxide, boron oxide and mixtures thereof, and an oxide selected from silicon oxide, germanium oxide, and mixtures thereof;
(b) maintaining the mixtures at a temperature of at least 140 °C until the crystals of said zeolite form; and
(c) recovering said crystals,
**characterized in that**
(d) the aqueous mixtures has a composition in terms of mole ratios of oxides falling in the ranges: YO₂/W₂O₃, 20:1 to 200:1; Q/YO₂, 0,15:1 to 0,50:1;
wherein Y is selected from silicon, germanium and mixtures thereof, W is selected from aluminium, gallium, iron, boron and mixtures thereof, and Q is an adamantane quarternary ammonium ion.

15. The method according to Claim 14 wherein the aqueous mixture has a composition in terms of mole ratios of oxides falling in the ranges: YO₂/W₂O₃, 20:1 to 200:1; Q/YO₂, 0.15:1 to 0.50:1; wherein Y is selected from silicon, germanium and mixtures thereof, W is selected from aluminum, gallium, iron, boron and mixtures thereof, and Q is an adamantane quaternary ammoniun ion.

16. A method according to Claims 14 or 15 wherein the adamantane quaternary ammonium ion is derived from an adamantane compound of the formula: 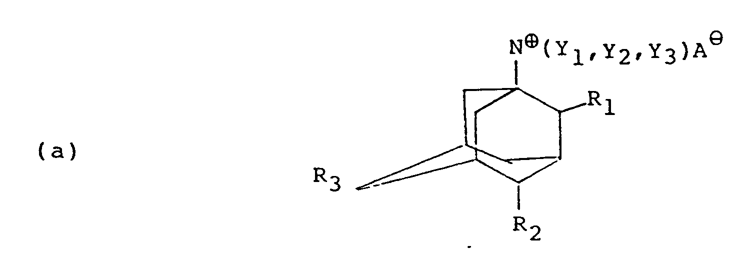 wherein each of Y₁ Y₂ and Y₃ independently is lower alkyl and A^{ϑ} is an anion which is not detrimental to the formation of the zeolite; and each of R₁, R₂ and R₃ independently is hydrogen, or lower alkyl; and 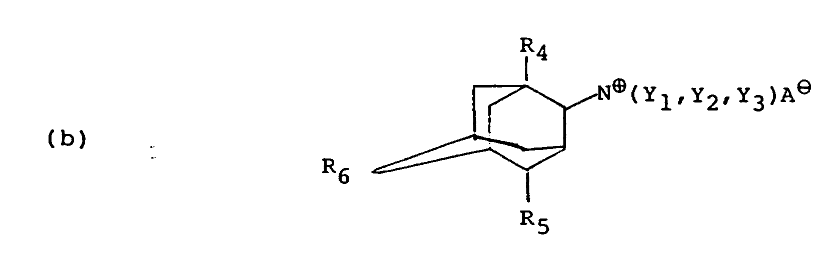 wherein each of R₄, R₅ and R₆ independently is hydrogen or lower alkyl; each of Y₁, Y₂ and Y₃ independently is lower alkyl; and A^{ϑ} is an anion which is not detrimental to the formation of the zeolite.

17. A method according to Claim 16 wherein in formula (a) each of Y₁, Y₂ and Y₃ independently is methyl or ethyl; A^{ϑ} is OH or halogen; and each of R₁, R₂, and R₃ is hydrogen; and in formula (b) each of Y₁, Y₂ and Y₃ independently is methyl or ethyl; A^{ϑ} is OH, or halogen; and each of R₄, R₅ and R₆ is hydrogen.

18. A method according to Claim 17 wherein Y₁, Y₂ and Y₃ are the same and each is methyl; and A^{ϑ} is OH, or I.

## Patentansprüche

1. Zeolith mit einem Molverhältnis eines Oxids, ausgewählt aus Siliziumoxid, Germaniumoxid und Mischungen davon, zu einem Oxid, ausgewählt aus Aluminiumoxid, Galliumoxid, Eisenoxid, Boroxid und Mischungen davon, das größer als etwa 20:1 ist, gekennzeichnet durch die folgenden Röntgenbeugungslinien:
| 2 Θ | d/n | I/Iₒ |
|---|---|---|
| 3,05 | 29,0 | 20 |
| 6,42 | 13,77 | 100 |
| 7,18 | 12,31 | 100 |
| 7,88 | 11,22 | 47 |
| 9,62 | 9,19 | 53 |
| 15,75 | 5,63 | 27 |
| 19,37 | 4,58 | 47 |
| 22,57 | 3,94 | 50 |
| 23,05 | 3,86 | 30 |
| 26,03 | 3,42 | 73 |
| 26,85 | 3,32 | 33 |

2. Zeolith mit einer Zusammensetzung in synthetisiertem und in wasserfreiem Zustand bezüglich der Molverhältnisse der Oxide, die wie folgt ist: (0,1 bis 2,0) Q₂O:(1,1 bis 2,0) M₂O:W₂O₃:-(größer als 20) YO₂, worin M ein Alkalimetall-Kation ist, W aus Aluminium, Gallium, Eisen, Bor und Mischungen davon ausgewählt ist, Y aus Silizium, Germanium und Mischungen daraus ausgewählt ist, und Q ein quarternäres Adamantanammoniumion ist, gekennzeichnet durch die folgenden Röntgenbeugungslinien:
| 2 Θ | d/n | I/Iₒ |
|---|---|---|
| 3,05 | 29,0 | 20 |
| 6,42 | 13,77 | 100 |
| 7,18 | 12,31 | 100 |
| 7,88 | 11,22 | 47 |
| 9,62 | 9,19 | 53 |
| 15,75 | 5,63 | 27 |
| 19,37 | 4,58 | 47 |
| 22,57 | 3,94 | 50 |
| 23,05 | 3,86 | 30 |
| 26,03 | 3,42 | 73 |
| 26,85 | 3,32 | 33 |

3. Zeolith nach Anspruch 1 oder 2, worin W Aluminium und Y Silizium ist.

4. Zeolith, hergestellt durch Wärmebehandlung des Zeoliths gemäß Anspruch 3 bei einer Temperatur von etwa 200°C bis 820°C.

5. Zeolith nach Anspruch 4 mit den Röntgenbeugungslinien von Tabelle 2.

6. Zeolith nach Anspruch 1 oder 2, bei dem das Molverhältnis von Siliziumoxid oder Germaniumoxid zu Aluminiumoxid, Galliumoxid, Eisenoxid oder Boroxid etwa 20:1 bis 200:1 beträgt.

7. Zeolith nach Anspruch 1 oder 2, bei dem das Molverhältnis des Siliziumoxids oder Germaniumoxids zu Aluminiumoxid, Galliumoxid, Eisenoxid oder Boroxid etwa 30:1 bis 100:1 beträgt.

8. Zeolith nach Anspruch 2, wobei das quarternäre Adamantanammoniumion aus einer Adamantanverbindung der folgenden Formel abstammt: 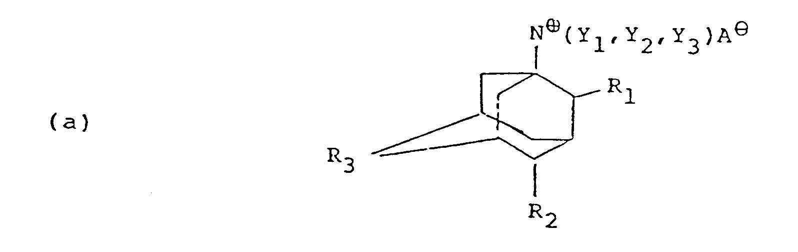 worin jedes Y₁ Y₂ und Y₃ unabhängig voneinander ein niederes Alkyl ist und A^{ϑ} ein Anion ist, das nicht nachteilig für die Bildung des Zeoliths ist; und jedes R₁, R₂ und R₃ unabhängig Voneinander ein Wasserstoff oder niederes Alkyl ist; und 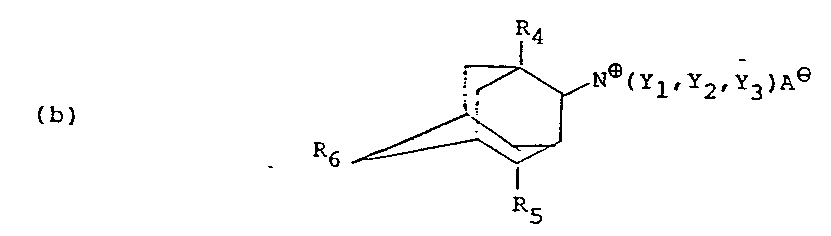 worin jedes R₄, R₅ und R₆ unabhängig voneinander ein Wasserstoff oder ein niederes Alkyl ist, jedes Y₁, Y₂ und Y₃ unabhängig Voneinander ein niederes Alkyl ist, und A^{ϑ} ein Anion ist, das nicht nachteilig für die Bildung des Zeoliths ist.

9. Zeolith nach Anspruch 8, wobei in Formel (a) jedes Y₁, Y₂ und Y₃ unabhängig voneinander ein Methyl odr Ethyl ist; A^{ϑ} OH oder ein Halogen ist; und jedes R₁, R₂ und R₃ Wasserstoff ist; und in Formel (b) jedes Y₁, Y₂ und Y₃ unabhängig voneinander ein Methyl oder Ethyl ist; A^{ϑ} OH oder ein Halogen ist; und jedes R₄, R₅ und R₆ ein Wasserstoff ist.

10. Zeolith nach Anspruch 9, wobei Y₁, Y₂ und Y₃ gleich sind und jedes ein Methyl ist; und A^{ϑ} OH ist oder I.

11. Zeolith nach Anspruch 1 oder 2, der einen Ionenaustausch mit Wasserstoff-, Ammonium-, Seltenerdenmetall-, Gruppe-IIA-Metall- oder Gruppe-VIII-Metallionen durchgemacht hat.

12. Zeolith nach Anspruch 1 oder 2, wobei Seltenerdenmetalle, Gruppe-IIA-Metalle, oder Gruppe-VIII-Metalle in dem Zeolith eingeschlossen sind.

13. Zeolithzusammensetzung, die den Zeolith gemäß Anspruch 1 oder 2 und eine anorganische Matrix umfaßt.

14. Verfahren zur Herstellung des Zeoliths der vorangegangenen Ansprüche, umfassend:
(a) Herstellung einer wässerigen Mischung, die Quellen eines quarternären Adamantanamoniumions, eines Oxids, ausgewählt aus Aluminiumoxid, Galliumoxid, Eisenoxid, Boroxid und Mischungen daraus, und eines Oxids, ausgewählt aus Siliziumoxid, Germaniumoxid, und Mischungen daraus, enthält;
(b) Halten der Mischungen bei einer Temperatur von wenigstens 140°C bis sich die Kristalle dieses Zeoliths bilden; und
(c) Gewinnen dieser Kristalle, gekennzeichnet dadurch,
(d) daß die wässerigen Mischungen eine Zusammensetzung bezüglich der Molverhältnisse der Oxide haben, die in den folgenden Bereichen liegt: YO₂/W₂O₃, 20:1 bis 200:1; Q/YO₂, o,15:1 bis 0,50:1; worin Y aus Silizium, Germanium und Mischungen daraus ausgewählt ist, W aus Aluminium, Gallium, Eisen, Bor und Mischungen daraus ausgewählt ist, und Q ein quarternäres Adamantanammoniumion ist.

15. Verfahren nach Anspruch 14, wobei die wässerige Mischung eine Zusammensetzung bezüglich der Molverhältnisse der Oxide hat, die in den Bereichen liegt: YO₂/W₂O₃, 20:1 bis 200:1; Q/YO₂, 0,15:1 bis 0,50:1, worin Y aus Silizium, Germanium und Mischungen daraus ausgewählt ist, W aus Aluminium, Gallium, Eisen, Bor und Mischungen daraus ausgewählt ist, und Q ein quarternäres Adamantanammoniumion ist.

16. Verfahren nach Anspruch 14 oder 15, wobei das quarternäre Adamantanammoniumion aus einer Adamantanverbindung der folgenden Formel stammt: 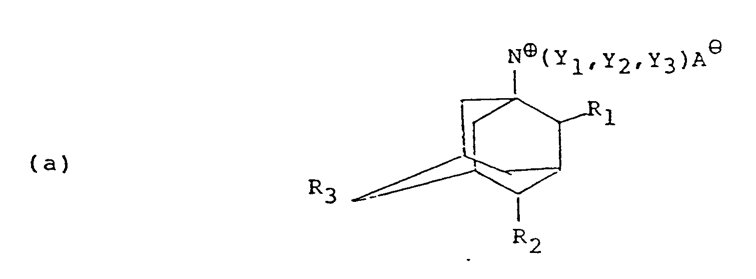 worin jedes Y₁ Y₂ und Y₃ unabhängig voneinander ein niederes Alkyl ist und A^{ϑ} ein Anion ist, das nicht nachteilig für die Bildung des Zeoliths ist; und jedes R₁, R₂ und R₃ unabhängig voneinander ein Wasserstoff, oder niederes Alkyl ist; und 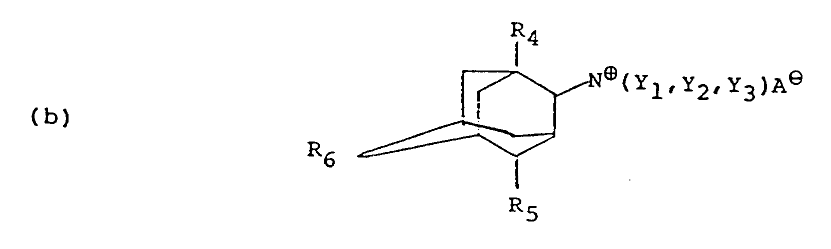 worin jedes R₄, R₅ und R₆ unabhängig voneinander Wasserstoff oder ein niederes Alkyl ist; jedes Y₁, Y₂ und Y₃ unabhängig voneinander ein niederes Alkyl ist; und A^{ϑ} ein Anion ist, das nicht nachteilig für die Bildung des Zeoliths ist.

17. Verfahren nach Anspruch 16, wobei in Formel (a) jedes Y₁, Y₂ und Y₃ unabhängig voneinander ein Methyl oder Ethyl ist; A^{ϑ} OH oder ein Halogen ist; und jedes R₁, R₂ und R₃ ein Wasserstoff ist; und in Formel (b) jedes Y₁, Y₂ und Y₃ unabhängig voneinander ein Methyl oder Ethyl ist; A OH oder ein Halogen ist; und jedes R₄, R₅ und R₆ ein Wasserstoff ist.

18. Verfahren nach Anspruch 17, wobei Y₁, Y₂ und Y₃ gleich sind und jedes ein Methyl ist; und A^{ϑ} OH oder I ist.

## Revendications

1. Zéolite ayant un rapport molaire d'un oxyde choisi entre l'oxyde de silicium, l'oxyde de germanium et leurs mélanges à un oxyde choisi entre l'oxyde d'aluminium, l'oxyde de gallium, l'oxyde de fer, l'oxyde de bore et leurs mélanges supérieur à environ 20:1,
caractérisée par les raies de diffraction des rayons X suivantes :
| 2 Θ | d/n | I/Iₒ |
|---|---|---|
| 3,05 | 29,0 | 20 |
| 6,42 | 13,77 | 100 |
| 7,18 | 12,31 | 100 |
| 7,88 | 11,22 | 47 |
| 9,62 | 9,19 | 53 |
| 15,75 | 5,63 | 27 |
| 19,37 | 4,58 | 47 |
| 22,57 | 3,94 | 50 |
| 23,05 | 3,86 | 30 |
| 26,03 | 3,42 | 73 |
| 26,85 | 3,32 | 33 |

2. Zéolite ayant, telle qu'elle est obtenue par synthèse et à l'état anhydre, la composition suivante exprimée par les rapports molaires des oxydes : (0,1 à 2,0)Q₂O:(1,1 à 2,0)M₂O:W₂O₃: (plus de 20) YO₂, M désignant un cation de métal alcalin, W étant choisi entre l'aluminium, le gallium, le fer, le bore et leurs mélanges, Y étant choisi entre le silicium, le germanium et leurs mélanges, Q représentant un ion ammonium quaternaire à fonction adamantane,
caractérisée par les raies de diffraction des rayons X suivantes :
| 2 Θ | d/n | I/Iₒ |
|---|---|---|
| 3,05 | 29,0 | 20 |
| 6,42 | 13,77 | 100 |
| 7,18 | 12,31 | 100 |
| 7,88 | 11,22 | 47 |
| 9,62 | 9,19 | 53 |
| 15,75 | 5,63 | 27 |
| 19,37 | 4,58 | 47 |
| 22,57 | 3,94 | 50 |
| 23,05 | 3,86 | 30 |
| 26,03 | 3,42 | 73 |
| 26,85 | 3,32 | 33 |

3. Zéolite suivant la revendication 1 ou 2, dans laquelle W représente l'aluminium et Y représente le silicium.

4. Zéolite préparée par traitement thermique de la zéolite suivant la revendication 3, à une température d'environ 200°C à 820°C.

5. Zéolite suivant la revendication 4, ayant les raies de diffraction des rayons X figurant sur le tableau 2.

6. Zéolite suivant la revendication 1 ou 2, dans laquelle le rapport molaire de l'oxyde de silicium ou de l'oxyde de germanium à l'oxyde d'aluminium, l'oxyde de gallium, l'oxyde de fer ou l'oxyde de bore est compris dans l'intervalle d'environ 20:1 à 200:1.

7. Zéolite suivant la revendication 1 ou 2, dans laquelle le rapport molaire de l'oxyde de silicium ou de l'oxyde de germanium à l'oxyde d'aluminium, l'oxyde de gallium, l'oxyde de fer ou l'oxyde de bore est compris dans l'intervalle d'environ 30:1 à 100:1.

8. Zéolite suivant la revendication 2, dans laquelle l'ion ammonium quaternaire à fonction adamantane est dérivé d'un adamantane de formule : 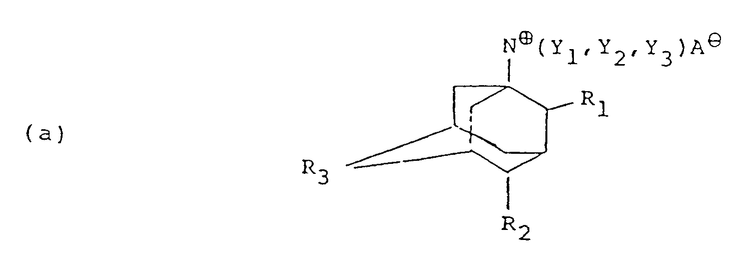 dans laquelle chacun des groupes Y₁, Y₂ et Y₃ représente, indépendamment, un groupe alkyle inférieur et A^{ϑ} représente un anion qui n'est pas néfaste pour la formation de la zéolite ; et chacun des groupes R₁, R₂ et R₃ représente, indépendamment, l'hydrogène ou un groupe alkyle inférieur ; ou 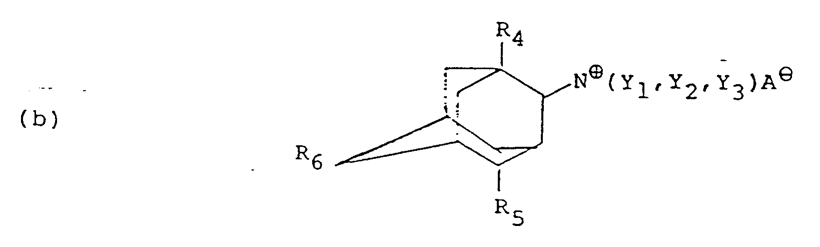 dans laquelle chacun des groupes R₄, R₅ et R₆ représente, indépendamment, l'hydrogène ou un groupe alkyle inférieur ; chacun des groupes Y₁, Y₂ et Y₃ représente, indépendamment, un groupe alkyle inférieur ; et A^{ϑ} représente un anion qui n'est pas néfaste pour la formation de la zéolite.

9. Zéolite suivant la revendication 8, dans laquelle, dans la formule (a), chacun des groupes Y₁, Y₂ et Y₃ représente, indépendamment, un groupe méthyle ou éthyle ; A^{ϑ} représente un groupe OH ou un halogène ; et chacun des groupes R₁, R₂ et R₃ représente l'hydrogène ; et, dans la formule (b), chacun des groupes Y₁, Y₂ et Y₃ représente, indépendamment, un groupe méthyle ou éthyle ; A^{ϑ} représente un groupe OH ou un halogène ; et chacun des groupes R₄, R₅ et R₆ représente l'hydrogène.

10. Zéolite suivant la revendication 9, dans laquelle Y₁, Y₂ et Y₃ sont identiques et représentent chacun un groupe méthyle ; et A^{ϑ} représente un groupe OH ou I.

11. Zéolite suivant la revendication 1 ou 2, qui a subi un échange d'ions avec des ions hydrogène, ammonium, des ions de métaux appartenant aux terres rares, de métaux du Groupe IIA ou de métaux du Groupe VIII.

12. Zéolite suivant la revendication 1 ou 2, dans laquelle les métaux appartenant aux terres rares, les métaux du Groupe IIA ou les métaux du Groupe VIII sont occlus dans la zéolite.

13. Composition de zéolite, comprenant la zéolite suivant la revendication 1 ou 2 et une matrice inorganique.

14. Procédé de préparation de la zéolite suivant les revendications précédentes, comprenant :
(a) la préparation d'un mélange aqueux contenant des sources d'un ion ammonium quaternaire à fonction adamantane, d'un oxyde choisi entre l'oxyde d'aluminium, l'oxyde de gallium, l'oxyde de fer, l'oxyde de bore et leurs mélanges / et d'un oxyde choisi entre l'oxyde de silicium, l'oxyde de germanium et leurs mélanges ;
(b) le maintien du mélange à une température d'au moins 140°C jusqu'à formation des cristaux de ladite zéolite ; et
(c) la séparation desdits cristaux, caractérisé en ce que
(d) le mélange aqueux possède une composition, en termes des rapports molaires des oxydes, comprise dans les intervalles : YO₂/W₂O₃, 20:1 à 200:1 ; Q/YO₂, 0,15:1 à 0,50:1 ; Y étant choisi entre le silicium, le germanium et leurs mélanges, W étant choisi entre l'aluminium, le gallium, le fer, le bore et leurs mélanges, et Q représentant un ion ammonium quaternaire à fonction adamantane.

15. Procédé suivant la revendication 14, dans lequel le mélange aqueux possède une composition, exprimée par les rapports molaires des oxydes, comprise dans les intervalles : YO₂/W₂O₃, 20:1 à 200:1 ; Q/YO₂, 0,15:1 à 0,50:1 ; Y étant choisi entre le silicium, le germanium et leurs mélanges, W étant choisi entre l'aluminium, le gallium, le fer, le bore et leurs mélanges, et Q représentant un ion ammonium quaternaire à fonction adamantane.

16. Procédé suivant la revendication 14 ou 15, dans lequel l'ion ammonium quaternaire à fonction adamantane est dérivé d'un adamantane de formule : 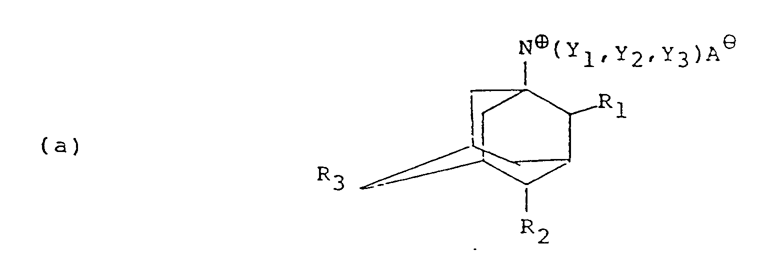 dans laquelle chacun des groupes Y₁, Y₂ et Y₃ représente, indépendamment, un groupe alkyle inférieur et A^{ϑ} représente un anion qui n'est pas néfaste pour la formation de la zéolite ; et chacun des groupes R₁, R₂ et R₃ représente, indépendamment, l'hydrogène ou un groupe alkyle inférieur ; ou 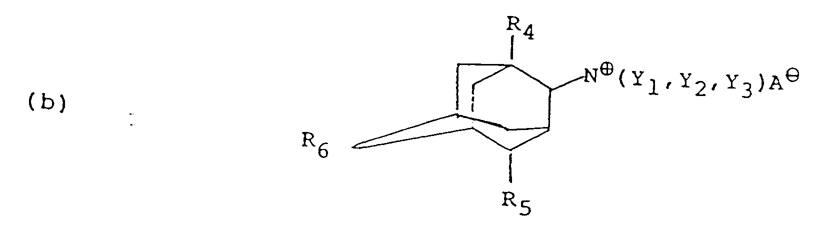 dans laquelle chacun des groupes R₄, R₅ et R₆ représente, indépendamment, l'hydrogène ou un groupe alkyle inférieur ; chacun des groupes Y₁, Y₂ et Y₃ représente, indépendamment, un groupe alkyle inférieur ; et A^{ϑ} représente un anion qui n'est pas néfaste pour la formation de la zéolite.

17. Procédé suivant la revendication 16, dans lequel, dans la formule (a), chacun des groupes Y₁, Y₂ et Y₃ représente, indépendamment, un groupe méthyle ou éthyle ; A^{ϑ} représente un groupe OH ou un halogène ; et chacun des groupes R₁, R₂ et R₃ représente l'hydrogène ; et, dans la formule (b), chacun des groupes Y₁, Y₂ et Y₃ représente, indépendamment, un groupe méthyle ou éthyle ; A^{ϑ} représente un groupe OH ou un halogène ; et chacun des groupes R₄, R₅ et R₆ représente l'hydrogène.

18. Procédé suivant la revendication 17, dans lequel Y₁, Y₂ et Y₃ sont identiques et représentent chacun un groupe méthyle ; et A^{ϑ} représente un groupe OH ou I.
